# EUROPEAN PATENT SPECIFICATION

(11) **EP 1 069 886 B1**
(45) Date of publication and mention of the grant of the patent: **12.11.2003**
(21) Application number: 99913784.7
(22) Date of filing: 08.03.1999
(51) Int. Cl.: A61K 9/107, A61K 7/00, A61K 47/00

(54) **USE OF A TOPICAL FORMULATION OF THE OIL-IN-WATER TYPE, COMPRISING GALACTOLIPID MATERIAL AS EMULSIFIER, FOR PROVIDING A PROLONGED EFFECT OF AN INCORPORATED ACTIVE SUBSTANCE**
VERWENDUNG VON TOPISCHEN FORMULIERUNGEN DES ÖL-IN-WASSER TYPS, ENTHALTEND GALAKTOLIPID MATERIAL ALS EMULSIONSMITTEL, UM EINE LÄNGER ANHALTENDE WIRKUNG EINES DARIN ENTHALTENEN AKTIVEN WIRKSTOFFS ZU VERMITTELN
USAGE D'UNE FORMULATION DE PRODUIT EN APPLICATION LOCALE DU TYPE EMULSION HUILE DANS L'EAU, COMPRENANT COMME EMULSIFIANT UN GALACTOLIPIDE, POUR OBTENIR L'EFFET PROLONGE D'UNE SUBSTANCE INCORPOREE

(30) Priority: 06.03.1998 SE 9800729
(43) Date of publication of application: 24.01.2001
(73) Proprietor: Lipocore Holding AB, 113 84 Stockholm (SE)
(72) Inventor: CARLSSON, Anders, S-112 38 Stockholm (SE); EK, Jenny, S-169 53 Solna (SE); HERSLÖF, Bengt, S-114 21 Stockholm (SE); NILSSON, Göran, S-112 52 Stockholm (SE)
(74) Representative: Larfeldt, Helene
(86) International application number: SE9900347
(87) International publication number: WO99044585

(56) References cited:
- EP-A1- 0 647 443
- WO-A1-95/20943

## Description

The present invention refers to a topical formulation of the oil-in-water emulsion type, in which a variety of pharmaceutical or cosmetic compounds can be incorporated, and which after application on the skin gives a prolonged local effect of the incorporated compound.

### BACKGROUND OF THE INVENTION

Dermatological formulations for topical administration, such as creams, lotions, ointments and gels, are used in pharmacy, medicine and cosmetics for curative and prophylac- tic treatment of different conditions. It is in general desirable that said formulation brings about a prolonged effect.

There are different areas where there is a continuous need of an improved, that is long lasting, topical treatment as exemplified below.
- People with very dry skin such as atopic dry skin and people who frequently are exposed to water and soap and thus often develop dry skin conditions need to apply a protective cream or ointment to their skin. Examples of people frequently exposed to water and soap are doctors and nurses who must wash their hands and face before examining patients, workers who are handling paints and grease and often need to use strong detergents to clean their hands, and, most common of all, home workers. For these and other categories of people having dry skin conditions a cream or lotion with an extended effect on skin smoothing and moisturising would be preferred.
- The use of hydrocortisone and other steroidal creams is very common in the treatment of local inflammatory conditions in the skin. The systemic absorption of the steroid potentially gives unwanted side-effects. A cream with a sustained release of the active steroid could increase the local effect and decrease the systemic absorption, a very much preferred therapeutic situation, especially in small children.
- The treatment of Athlete's foot or other fungal infections with topical antifungal or of many skin infec- tions with antibiotics or antivirals requires twice or three times daily applications of the cream or gel to be effective. A once-daily formulation would certainly improve compliance, effectiveness, as well as comfort during treatment.
- The treatment of any other skin condition, including psoriasis, eczemas, other inflammatory disorders, cancers, precancerous conditions, ageing, wrinkling, ultraviolet radiation damage and any other condition which may respond to a topically applied therapeutic agent.

Preferred topical formulations are creams and lotions, that is typically oil-in-water emulsions which spread readily on the skin, leave no detectable residue and adhere to the treated area without being tacky. Said emulsions normally consist of an oil phase, an aqueous phase and an emulsifier. Ointments, which mainly comprises an oil phase, are greasy and form a greasy film on the skin preventing moisture loss. Gels which might be liposomal preparations do not contain any oil. Topical preparations of the oil-in-water emulsion type are generally more appreciated by the user from a cosmetic point of view, but have not previously been claimed to give any extended effect of incorporated substances of dermatological or cosmetological interest. From a dermatological standpoint oil-in-water emulsion type formulations are often preferred, particularly if the number of ingredients can be reduced to a minimum.

### PRIOR ART

Highly structured vehicles, such as inversed hexagonal and cubic liquid crystals, may exhibit sustained-release properties, either by binding the water or by stiffening the amphiphilic film within the formulation, see Osborne, D.W., et al. in Drugs and the Pharmaceutical Sciences, Swarbrick J.(ed.), Vol. 42 (1990), pp. 374-379. Drug formulations containing liposomes for topical use may give a sustained local effect of the incorporated compound, see Korting, H.C., et al. in J. Am. Acad. Dermatol., Vol. 25 (1991), pp. 1068-1071. The topical drug delivery systems described are, however, far more complicated lipid preparations than a topical cream of the oil-in-water emulsion type. For reasons of stability of topical liposomal systems, most authors have proposed a gel base. Gel formulations are, however, more likely to produce side effects than cream or ointment preparations.

WO 95/03784, Insite Vision Inc., discloses a cross-linked polymeric medicament delivery system containing an interactive agent associated with the polymer, which is said to slow release of medicament out of the system. The system can be used in dermal formulations but is particularly useful as topical ophthalmic delivery systems. This invention does not relate to any slow release effects of the cream, but on polymeric systems included in the cream. The slow release effects in this system can be ascribed to the polymeric system.

Topical creams of the oil-in-water emulsion type have not previously been reported as having potential sustained release properties. However, there is a need for topical sustained release formulations, such as oil-in-water emulsions, which are uncomplicated with respect to compositional design as well as manufacturing. Furthermore, less complicated formulations have a major advantage in that they are less likely to cause irritant or hypersensitivity reactions and hence to be more acceptable as skin care preparations for therapeutic or cosmetic use.

WO 95/20943, Karlshamns LipidTeknik AB, discloses an oil-in-water emulsion comprising 0.01-50 % by weight of a galactolipid material as an emulsifier. Said emulsion is said to be useful as a carrier for active substances in a pharmaceutical composition but also in nutritional, cosmetic, food and agricultural products. The emulsions do not exhibit any unpleasant odour or taste and are stable towards oxidation. There is, however, nothing stated about an optional sustained effect.

### DESCRIPTION OF THE INVENTION

The present invention relates to the use of an oil-in-water emulsion for topical application to the skin comprising an emulsifier, an oil phase, and an aqueous phase, into which cosmetic or pharmaceutical substances can be incorporated for the local treatment of various skin conditions and disorders.

It has surprisingly been found that a topical cream or lotion of the oil-in-water emulsion type, in which a galactolipid material is used as the emulsifier, and into which a variety of pharmaceutical or cosmetic compounds can be incorporated, after application on the skin gives a sustained local effect of the incorporated compound.

The present invention refers to the use of an oil-in-water emulsion, comprising an oily material, an aqueous phase and a galactolipid material as an emulsifier, as a carrier for a topical formulation for providing a prolonged local effect of an incorporated pharmaceutically or cosmetically active substance.

Especially the invention refers to the use of a topical formulation, which can be a cream or a lotion, comprising 0.1-50 % by weight oily material, preferably 1-40 %, and 0.5-20 % by weight emulsifier.

No particular limitation is imposed on the oily material, that is the non-polar lipid material, of the formulation. Examples are vegetable oils, animal oils, fatty acids, synthetic oils, mineral oils, natural and synthetic glycerides, sterol esters, fatty alcohols, and other substances, including lipophilic drugs, obvious to a person skilled in the art, which can be emulsified using a polar lipid emulsifier.

Preferred oily materials to be emulsified are any fatty acid or a derivative thereof, such as vegetable oils of all types, such as oils from the seeds and beans of soybean, sunflower, rapeseed (canola), palm, corn, evening primrose, borage, groundnut, sesame, and similar.

There are also synthetic or semi-synthetic glycerides, propanediol derivatives, cholesteryl esters, other esters and other appropriata lipid materials. Another oily material for the emulsion is a medium-chain triacylglycerol (MCT) oil.

There are also many lipids such as free fatty acids, mono-, di- and triacylglycerols, phospholipids, cholesterol esters and lipids and oils of many other types which have therapeutic actions in themselves, such as tea tree oil, and which may be advantageously formulated in the form of a topical cream or optionally lotion. In this case the therapeutically active substance is the oily material, which can also have other bioactive properties.

The emulsifier according to the invention should be a galactolipid based material. Galactolipids can be defined as glycosylglycerides based on galactose and are well known constituents of plant cell membranes. The most important classes of these contain one to four sugars linked glycosidically to diacylglycerol. The two most abundant classes contain one and two galactose units, respectively, and are commonly known as mono- and digalactosyldiacylglycerol, MGDG and DGDG. Galactolipids, primarily DGDG and DGDG-rich materials, have been investigated and found to be a surface active material of interest in industrial application such as food, cosmetics, and pharmaceutical applications.

Synthetic diglycosyldiacylglycerols based on galactose, optionally in combination with other monosaccharide units, such as glucose, semi-synthetic, and natural glycosylglycerides, isolated from any source, can be used in accordance with the invention.

An intrinsic beneficial feature of the galactolipids is the galactose units comprising the polar head group in each lipid molecule, which may sterically stabilise the emulsion droplets in an emulsion. The galactose groups may also interact strongly with water and other polar substances, such as a water-soluble drug or a excipient, added to the emulsion.

WO 95/20943 describes the use of DGDG-rich material, a galactolipid material, as an emulsifier in oil-in-water emulsions. Said galactolipid material was prepared from cereals by extraction of the lipids with ethanol and a subsequent purification on a chromatographic column to pure DGDG or a DGDG-rich fraction of polar lipids. The galactolipid emulsifier consists of at least 50 % digalactosyldiacylglycerols and a remainder of other polar lipids and can be used as the galactolipid emulsifier of the invention, preferably in an amount of 1.0-5.0 % by weight. The galactolipid material for instance consists of 70-80 % DGDG and 20-30 % other polar lipids.

According to a preferred embodiment of the invention the galactolipid emulsifier consists of 50-70 % digalactosyldiacylglycerols and 30-50 % other polar lipids. This material is manufactured by Scotia LipidTeknik AB, Stockholm, as CPL®-Galactolipid (registered trade mark owned by Scotia Holdings plc). A preferred topical formulation of the invention comprises CPL®-Galactolipid as the galactolipid material.

WO 97/11141 describes a method for producing a fractionated vegetable oil which is characterised in containing 10-90 % by weight of polar lipids, preferably 20-75 %, and a remainder of non-polar lipids. Said fractionated vegetable oil can also be used as the galactolipid emulsifier of the invention, preferably in an amount of 2.0-10.0 % by weight. The fractionated vegetable oil preferably contains more than 5 % by weight, preferably more than 20 %, glycolipids and preferably more than 3 % by weight, preferably more than 15 %, DGDG.

According to a preferred embodiment of the invention the galactolipid material consists of 40-60 % polar lipids and a remainder of non-polar lipids. A fractionated oat oil of this composition consisting of a wide range of polar and amphiphilic lipids in a continuous triglyceride phase is manufactured by Scotia LipidTeknik AB, Stockholm, as Galactolec™. A preferred topical formulation comprises Galactolec™ as the galactolipid material.

The galactolipid based emulsifier is a safe and non-toxic material for human and veterinary use. It is also an environmentally friendly material.

Topical formulations, such as creams and lotions, are prepared by using a polar lipid emulsifier either as the sole emulsifier or in combination with other amphiphilic compounds, that is co-surfactants. The formulation may also comprise optional additives known in the art for improving different aspects of the composition, such as thickening agents, preservatives, antioxidants, fragrance and the like.

The creams according to the invention are characterised by having excellent cosmetic properties. Furthermore, they contain a minimum number of ingredients, without any stabilising ingredients known to give irritation or sensitisation of the skin. Despite the low numbers of ingredients the creams are extremely stable, with shelf lives of several years.

The active substances can be either water soluble or oil soluble or amphiphilic, and can be any type of pharmaceutical or cosmetological ingredient suitable for topical preparations, such as moisturising agents, e.g. glycerol, propylene glycol, urea, vitamins, e.g. retinol and tocopheryl esters, antiinflammatories, e.g. glucocorticosteroids such as hydrocortisone, hydrocortisone butyrate, chlobetasol, triamcinolone, fluticasone, momethasone and betamethasone, antibiotics, e.g. erythromycin, antivirals, e.g. acyclovir, antifungals, e.g. miconazole, antiseptics, e.g. cetrimide, agents for treating acne, e.g. tretinoin, benzylperoxide, psoriasis, e.g. dithranol and calcipotriol, senile pruritus, dry skin and wrinkles, cancer and pre-cancerous conditions, such as active keratosis, and UV protecting agents to be included in suntan creams and lotions.

Topical creams according to the invention are prepared by conventional methods. For example, a cream with 20 % by weight of oil is prepared by adding the emulsifier to a triacylglycerol oil. The oil phase may also contain oil-soluble additives such as antioxidants and fragrance. The total emulsifier concentration is 1.5 % by weight. The oil phase is then gently mixed. The continuous phase may be pure water or an aqueous solution containing water-soluble additives such as glycerol, preservatives and buffers. A water-soluble active compound, such as glycerol as a moisturiser, may then be added to the aqueous phase; consequently, an oil-soluble compound such as 13-hydroxy-9,11-octadecadienoic acid (13-HODE) is added to the oil phase. Hydrocortisone, an anti-inflammatory drug which is insoluble in both water and oil, may be dispersed in either the aqueous phase or the oil phase. Alternatively, the drug may also be added to the final cream in an extemporaneous preparation. If necessary, the pH of the aqueous phase is adjusted. The oil phase as well as the aqueous phase are preheated to 70°C and then the oil phase is added to the aqueous phase under high-shear mixing. The pre-emulsion is then subjected to homogenisation at 200 psi. After cooling, the cream is transferred to suitable containers.

The invention also refers to the use of a topical formulation of the invention, wherein the incorporated compound is a moisturising compound for the preparation of a medicament for prophylaxis or treatment of atopic dermatitis.

Formulations, that is creams and lotions, having the following, preferred compositions can be prepared accordingly:
Topical cream base giving an incorporated substance a prolonged effect, comprising in % by weight

| | |
|---|---|
| Oily material | 10.0-30.0 % |
| Galactolipid emulsifier | 0.5-5 % |
| Thickener | 2.0-10.0 % |
| Preservative | 0.1-1.0 % |
| Water | ad 100 % |

Topical formulation having a prolonged moisturising effect, comprising in % by weight

| | |
|---|---|
| Glycerol | 1.0-5.0 % |
| Oily material | 10.0-30.0 % |
| Galactolipid emulsifier | 0.5-5 % |
| Thickener | 2.0-10.0 % |
| Preservative | 0.1-1.0 % |
| Water | ad 100 % |

Topical formulation having a prolonged anti-inflammatory effect, comprising in % by weight

| | |
|---|---|
| Hydrocortisone | 0.5-1.5 % |
| Oily material | 10.0-30.0 % |
| Galactolipid emulsifier | 0.5-5 % |
| Thickener | 2.0-10.0 % |
| Preservative | 0.1-1.0 % |
| Water | ad 100 % |

Topical formulation having a prolonged anti-inflammatory effect, comprising in % by weight

| | |
|---|---|
| Betamethasone | 0.01-0.5 % |
| Oily material | 10.0-30.0 % |
| Galactolipid emulsifier | 0.5-5 % |
| Thickener | 2.0-10.0 % |
| Preservative | 0.1-1.0 % |
| Water | ad 100 % |

Topical formulation having a prolonged anti-psoriatic effect, comprising in % by weight

| | |
|---|---|
| 13-hydroxy-linoleic acid | 0.001-0.1 % |
| Oily material | 10.0-30.0 % |
| Galactolipid emulsifier | 0.5-5 % |
| Thickener | 2.0-10.0 % |
| Preservative | 0.1-1.0 % |
| Water | ad 100 % |

Different topical formulations with various non-polar oils as the cream base were formulated as described in Examples 1-7. Typical batch sizes are 0.5 to 1 kg. All concentrations are expressed in percent by weight.

### EXAMPLES OF FORMULATIONS

### Example 1. Moisturising cream

| Oil phase: | | |
|---|---|---|
| CPL®-Soybean oil | 20.0 % | Oily material |
| Cetostearyl alcohol | 7.0 % | Thickener |
| Glyceryl monostearate/citrate | 2.0 % | Thickener |
| Emulsifier: CPL®-Galactolipid | 1.5 % | |

| Aqueous phase: | | |
|---|---|---|
| Glycerol | 2.0 % | Moisturiser |
| Methyl-p-hydroxybenzoate | 0.54 % | Preservative |
| Propyl-p-hydroxybenzoate | 0.06 % | Preservative |
| Water | ad 100 % | |

The oil and CPL®-Galactolipid were mixed in a beaker and then stirred with a magnetic stirrer until the galactolipid material had dispersed, that is for 30-60 min. The aqueous phase was mixed in another beaker and stirred with a magnetic stirrer. When the oil phase was homogeneous glyceryl monostearate/citrate and cetostearyl alcohol were added. The oil phase and the aqueous phase were both heated to 65-70°C while stirring. The warm oil phase was added to the warm aqueous phase during high-shear mixing (Polytron PT-MR 3000). After addition of the oil phase the pre-emulsification (high-shear mixing) continued for 2 minutes at 15,000 rpm. The pre-emulsion was then homogenised 2 times at 200 psi in an Ultrasonic homogeniser (Branson Minisonic 4). The cream was allowed to cool in a water bath.

### Example 2. Moisturising lotion

| Oil phase: | | |
|---|---|---|
| CPL®-Evening Primrose oil | 12.0 % | Oily material |
| Cetostearyl alcohol | 2.0 % | Thickener |
| Glyceryl monostearate/citrate | 2.0 % | Thickener |
| Ascorbyl palmitate | 0.02 % | Antioxidant |

| Emulsifier: | | |
|---|---|---|
| CPL®-Galactolipid | 1.0 % | |

| Aqueous phase: | | |
|---|---|---|
| Glycerol | 2.0 % | Moisturiser |
| Methyl-p-hydroxybenzoate | 0.54% | Preservative |
| Propyl-p-hydroxybenzoate | 0.06% | Preservative |
| Fragrance | 0.1 % | |
| Water | ad 100 % | |

The lotion was prepared in the same way as the cream in Example 1, that is CPL®-Evening Primrose Oil, CPL®-Galactolipid and ascorbyl palmitate were mixed in a beaker and stirred until the galactolipid material had dispersed properly, that is for 30-60 minutes. The rest of the ingredients was added to the oil phase which was then heated to 70°C. The aqueous phase was prepared in another beaker and heated to 70°C. The oil phase was added to the aqueous phase during high-shear mixing. After addition of the oil phase the high-shear mixing, that is pre-emulsification, continued for 2 min at 15,000 rpm. The pre-emulsion was homogenized twice at 200 psi in an Ultrasonic homogeniser (Branson Minisonic 4). The lotion was allowed to cool in a water bath. The fragrance was added to the cool, that is 35°C, lotion.

### Example 3. Moisturising cream

| Oil phase: | | |
|---|---|---|
| CPL®-Evening Primrose oil | 20.0 % | Oily material |
| Cetostearyl alcohol | 7.0 % | Thickener |
| Glyceryl monostearate/citrate | 2.0 % | Thickener |
| Ascorbyl palmitate | 0.02% | Antioxidant |

| Emulsifier: | | |
|---|---|---|
| Galactolec™ | 3.0 % | |

| Aqueous phase: | | |
|---|---|---|
| Glycerol | 2.0 % | Moisturiser |
| Methyl-p-hydroxybenzoate | 0.63% | Preservative |
| Propyl-p-hydroxybenzoate | 0.07% | Preservative |
| Water | ad 100 % | |

The cream was prepared as described in Example 1.

The cream had the following appearance in the microscope. Small regular to irregular droplets of uniform size evenly distributed in the sample. The average droplet size, estimated by comparison with a ruler installed in the microscope, was found to be in the range of 5-10 µm.

### Example 4. Cream base

| Oil phase: | | |
|---|---|---|
| Olive oil | 20.0 % | Oily material |
| Cetostearyl alcohol | 7.0 % | Thickener |
| Glyceryl monostearate | 2.0 % | Thickener |

| Emulsifier: | | |
|---|---|---|
| CPL®-Galactolipid | 1.0 % | |

| Aqueous phase: | | |
|---|---|---|
| Methyl-p-hydroxybenzoate | 0.54 % | Preservative |
| Propyl-p-hydroxybenzoate | 0.06 % | Preservative |
| Tri-sodium citrate dihydrate | 0.035% | pH-modifier |
| Citric acid (aq.) | q.s. pH 3.5 | pH-modifier |
| Water | ad 100 % | |

The cream was prepared as described in Example 1.

### Example 5. Cream base

| Oil phase: | | |
|---|---|---|
| Medium-chain triglyceride oil | 10.0 % | Oily material |
| Cetostearyl alcohol | 7.0 % | Thickener |
| Glyceryl monostearate | 2.0 % | Thickener |

| Emulsifier: | | |
|---|---|---|
| CPL®-Galactolipid | 1.0 % | |

| Aqueous phase: | | |
|---|---|---|
| Methyl-p-hydroxybenzoate | 0.18 % | Preservative |
| Propyl-p-hydroxybenzoate | 0.02 % | Preservative |
| Water | ad 100 % | |

The cream was prepared as described in Example 1.

### Example 6. Anti-inflammatory cream

| Oil phase: | | |
|---|---|---|
| Hydrocortisone | 1.0 % | Active substance |
| CPL®-Evening Primrose oil | 20.0 % | Oily material |
| Cetostearyl alcohol | 7.0 % | Thickener |
| Glyceryl monostearate | 2.0 % | Thickener |
| Ascorbyl palmitate | 0.02 % | Antioxidant |

| Emulsifier: | | |
|---|---|---|
| CPL®-Galactolipid | 1.5 % | |

| Aqueous phase: | | |
|---|---|---|
| Glycerol | 2.0 % | Moisturiser |
| Methyl-p-hydroxybenzoate | 0.63 % | Preservative |
| Propyl-p-hydroxybenzoate | 0.07 % | Preservative |
| Water | ad 100 % | |

Hydrocortisone was added to the mixture of oil and galactolipid. Otherwise the cream was prepared as described in Example 1.

### Example 7. Anti-inflammatory cream

| Oil phase: | | |
|---|---|---|
| Betamethasone, dipropionate | 0.05 % | Active substance |
| CPL®-Evening Primrose oil | 20.0 % | Oily material |
| Cetostearyl alcohol | 7.0 % | Thickener |
| Glyceryl monostearate | 2.0 % | Thickener |
| Ascorbyl palmitate | 0.02 % | Antioxidant |

| Emulsifier: | | |
|---|---|---|
| CPL®-Galactolipid | 1.5 % | |

| Aqueous phase: | | |
|---|---|---|
| Glycerol | 2.0 % | Moisturiser |
| Methyl-p-hydroxybenzoate | 0.63 % | Preservative |
| Propyl-p-hydroxybenzoate | 0.07 % | Preservative |
| Water | ad 100 % | |

A comparable cream is obtained if betamethasone, dipropionate 0.05 % is replaced by betamethasone, valerate 0.1 %.

### Example 8. Anti-psoriatic cream

| Oil phase: | | |
|---|---|---|
| 13-HODE | 0.01 % | Active substance |
| CPL®-Evening Primrose oil | 20.0 % | Oily material |
| Cetostearyl alcohol | 7.0 % | Thickener |
| Glyceryl monostearate | 2.0 % | Thickener |
| Ascorbyl palmitate | 0.02 % | Antioxidant |

| Emulsifier: | | |
|---|---|---|
| CPL®-Galactolipid | 1.5 % | |

| Aqueous phase: | | |
|---|---|---|
| Methyl-p-hydroxybenzoate | 0.63 % | Preservative |
| Propyl-p-hydroxybenzoate | 0.07 % | Preservative |
| Water | ad 100 % | |

A small amount, about 5 %, of the oil mixture was added to 13-HODE (13-hydroxy-linoleic acid, from Scotia Pharmaceuticals Ltd, Carlisle). This mixture was not heated like the rest of the oil phase and was added separately during the pre-emulsification step. Otherwise the cream was prepared as in Example 1.

### EXPERIMENTAL TESTS

### Tests of skin smoothing and moisturising properties.

The aim of the studies was to evaluate the moisturising and smoothing properties of creams of the invention after use twice daily for 14 days. Twenty healthy female volunteers aged 18 to 60 years were studied.

The test creams had the following compositions:

| | Cream A | Cream B | Cream C |
|---|---|---|---|
| Oil phase: | | | |
| CPL®-Evening Primrose oil | 20.0 % | 20.0 % | 20.0 % |
| Cetostearyl alcohol | 7.0 % | 7.0 % | 7.0 % |
| Ascorbyl palmitate | 0.02 % | 0.02 % | 0.02 % |
| | | | |

| Emulsifier: | | | |
|---|---|---|---|
| CPL®-Galactolipid | 0.75 % | 0.75 % | 1.5 % |
| | | | |

| Aqueous phase: | | | |
|---|---|---|---|
| Glycerol | - | 2.0 % | 2.0 % |
| Methyl-p-hydroxybenzoate | 0.54 % | 0.54 % | 0.63 % |
| Propyl-p-hydroxybenzoate | 0.06 % | 0.06 % | 0.07 % |
| Water | ad 100 % | ad 100 % | ad 100 % |

All creams were prepared in the following way: The CPL®-Evening Primrose oil, CPL®-Galactolipid and ascorbyl palmitate were mixed in a beaker and then stirred with a magnetic stirrer until the galactolipid was completely dispersed, that is for 30-60 min. The aqueous phase was mixed in another beaker and stirred with a magnetic stirrer. When the oil phase was homogeneous, cetostearyl alcohol was added. The oil phase and the aqueous phase were both heated to 55°C while stirring. The warm oil phase was added to the warm aqueous phase during high-shear mixing (Polytron PT-MR 3000). After addition of the oil phase the pre-emulsification continued for 2 min at 15,000 rpm. The pre-emulsion was then homogenised 6 times at 200 psi in an Ultrasonic homogeniser (Branson Minisonic 4). The cream was allowed to cool in a water bath.

On the first day of the study the subjects were instructed as to the proper manner of application of the products. The creams were then applied by the subjects at home once in the morning and once in the evening as part of the daily body care routine.

An amount approximating the usual applied amount of skin care cream (one fingertip full, approximately 0.2 ml) was taken from the respective container, applied to the test fields noted on the container and rubbed in with the finger. The test fields were not marked during the application period. In order to locate the test fields, the inside of the forearm was optically divided into thirds. The middle third was defined as the lower test field and the upper third as the upper test field. An area the width of two fingers was left free between the two test fields on the underarm. A field on the inside of the upper arm served as the upper test field. The subjects were given a stencil to simplify locating the boundary between the lower and middle field on each arm.

The subjects were instructed that the finger used to apply the creams had to be carefully cleaned with a dry cloth between applications to avoid mixing of the test preparations.

Skin moisture was assessed using a device for determining the capacitance of the skin surface (Corneometer CM 820, Courage & Khazaka, Cologne). The capacity of a conductor (the more or less moist stratum corneum on the skin surface) to store an electric charge is recorded using this method. The instrument probe was held onto the skin without pressing for a brief, defined interval. Five measurements were made per test field. The mean of the five measurements was recorded on-line.

Following the measurement of skin moisture, a negative replica of the skin was made using 2-component silicone rubber impression material (Xantopren® L, Fa. Bayer Dental, Leverkusen, Germany). The subjects laid the stretched but relaxed arms on special arm rests with the inner surface facing upwards. A surface of approximately 8 x 8 cm in the centre of the test fields was thinly covered with the impression mass mixed with hardener. Approximately 3 min were required for setting. The replicas were peeled off after 8 min. Labels were pressed into the lower edge of the hardening mass. These serve for identification as well as marking of the alignment.

The surface of the silicone replicas was scanned using a Hommel-Tester T2000 (Hommelwerke, Schwenningen, Germany). The path and speed of scanning were controlled over the software. The surface was characterised by the roughness parameter R_{Z(DIN)}. Each replica was measured in a star-shaped fashion in 12 directions (30° angles).

Skin moisture was measured and replicas taken immediately before the first application of treatments (baseline) and on study days 15, 16 and 17. The measurements on day 15 were performed 12 to 16 hours after the last application. The measurements on day 16 and 17 were performed 36 to 40 h and 60 to 64 h, respectively, after the last application. The silicone replicas were made directly following the corneometer measurements. The results are presented in Table 1 and 2.

Cream A did not lead to any improvement at all in skin moisture. The incorporation of an active moisturising agent (glycerol) in Cream B resulted in a clearly demonstrated moisturising effect as expected. Unexpectedly though, the effect was also long lasting.

**Table 1.**

| Skin moisturisation. | | |
|---|---|---|
| | Comparison | Moisturisation |
| Cream A (no active) | day 0 vs. day 15 | -1.3 % |
| | day 0 vs. day 16 | -0.9 % |
| Cream B (glycerol) | day 0 vs. day 15 | +6.3 %** |
| | day 0 vs. day 16 | +7.3 %** |
| Cream C (glycerol) | day 0 vs. day 15 | +16.9 %** |
| | day 0 vs. day 16 | +12.7 %** |
| | day 0 vs. day 17 | +6.6 %** |

| | | |
|---|---|---|
| *= p<0.1 ** = p<0.05 | | |

**Table 2.**

| Skin roughness. | | |
|---|---|---|
| | Comparison | Smoothing |
| Cream C (glycerol) | day 0 vs. day 15 | +6.3 %* |
| | day 0 vs. day 16 | +6.6 %** |
| | day 0 vs. day 17 | +3.3 %* |

| | | |
|---|---|---|
| *= p<0.1 | | |
| ** = p<0.05 | | |

The sustained effect found for Cream B was even more pronounced for Cream C which contained a higher content of the galactolipid based emulsifier. Conventional creams containing glycerol have not been reported to exhibit any sustained moisturising effect at all. The results presented in Table 1 and 2 clearly and surprisingly demonstrate a moisturising as well as a smoothing effect which last for at least three days after the last application.

The test of skin smoothing and moisturising properties of creams was repeated with slightly different cream compositions. The test creams had the following compositions:

| | Cream D |
|---|---|
| Oil phase: | |
| CPL®-Evening Primrose oil | 20.0 % |
| Cetostearyl alcohol | 7.0 % |
| Glyceryl stearate | 2.0 % |
| Ascorbyl palmitate | 0.02 % |
| | |

| Emulsifier: | |
|---|---|
| CPL®-Galactolipid | 1.5 % |
| | |

| Aqueous phase: | |
|---|---|
| Glycerol | 2.0 % |
| Methyl-p-hydroxybenzoate | 0.63 % |
| Propyl-p-hydroxybenzoate | 0.07 % |
| Water | ad 100 % |
| | |

In creams E, F and G the CPL® - Evening primrose oil was replaced by the same amount, i.e. 20 % of soybean oil, MCT oil and liquid paraffin oil, respectively. All other ingredients and the amounts of each were as in cream D.

Obviously the prolonged moisturising effect is dependent of the choice of oil as shown in Table 3 below. However, all four creams based on the oil-in-water emulsion type, described in the present invention, have a general ability of prolonging the moisturising effect compared to commercially available creams.

**Table 3.**

| Skin moisturisation | | |
|---|---|---|
| | Comparison | Moisturisation |
| Cream D | day 0 vs. day 15 | +8.4 %** |
| (Evening primrose oil) | day 0 vs. day 16 | +10.9 %** |
| | day 0 vs. day 17 | +6.0 %** |
| | | |
| Cream E | day 0 vs. day 15 | +6.7 %** |
| (Soybean oil) | day 0 vs. day 16 | +4.3 % |
| | day 0 vs. day 17 | +1.7 % |
| | | |
| Cream F | day 0 vs. day 15 | +8.8 %** |
| (MCT oil) | day 0 vs. day 16 | +6.3 %** |
| | day 0 vs. day 17 | +5.8 %* |
| | | |
| Cream G | day 0 vs. day 15 | +7.2 %** |
| (Liquid paraffin oil) | day 0 vs. day 16 | +6.5 %** |
| | day 0 vs. day 17 | +2.4 % |

| | | |
|---|---|---|
| * = p<0.1 | | |
| ** = p<0.05 | | |

### A consumer test

Thirty human volunteers participated in a consumer test of cream D described above. All subjects were regular users of emollients and moisturisers; eighteen subjects because of having atopic dry skin and twelve subjects because of necessary frequent exposure to detergents and water, e.g. during work at hospitals.

All subjects received one tube containing 100 ml of the cream and a questionnaire to fill in prior to, during and after having used the cream for two days.

The questionnaire was divided into six parts. The first part covered the background, sex, date of birth, the reason for and the frequency of using emollients etc. Parts two, three, four and five covered questions related to: the immediate reaction, 5-10 minutes later, after washing of hands, and after two days of using the cream, respectively. Part six covered "Further comments". In parts two, three, four and five, the question raised was, "To what extent do you agree with the following statements?" The extent of agreement could be given a score between 0 and 10, where 0 meant "No, not at all" and a score of 10 meant "Yes, definitely". For practical reasons the score results were grouped together according to the following:
0-2: No, not at all
3-7: Yes, to a certain degree
8-10: Yes, definitely

The moisturising effect was found to be more long lasting than what is normally experienced with this type of products. It is also clear from the results presented in Table 4 that washing the skin is not detrimental to the effect, which is normally the case when using emollients and moisturisers. It makes it much easier to keep the skin smooth and supple and to avoid dryness. The cream was not found to be irritating to dry and sensitive skin. It seems to be very well tolerated also by persons with atopic dry skin.

**Table 4.**

| | | | |
|---|---|---|---|
| To what extent do you agree with the following statements? | | | |

| No. of subjects giving the score | | | |
|---|---|---|---|
| Extent of agreement | 0-2 | 3-7 | 8-10 |
| Statement | | | |
| *Immediate reaction* | | | |
| "The cream is easily absorbed into the skin" | 3 | 13 | 14 |
| "The cream is greasy on my skin" | 10 | 14 | 5 |
| "The odour of the cream is unpleasant" | 22 | 7 | 1 |
| "The cream irritates the skin" | 30 | 0 | 0 |
| "The skin feels smooth and supple" | 1 | 4 | 25 |
| | | | |

| *5-10 minutes later* | | | |
|---|---|---|---|
| "The cream is greasy on my skin" | 24 | 4 | 2 |
| "The odour of the cream is unpleasant" | 22 | 5 | 1 |
| "The skin feels smooth and supple" | 0 | 6 | 24 |
| "The skin is dry" | 25 | 4 | 1 |
| | | | |

| *After washing of hands (with soap and water)* | | | |
|---|---|---|---|
| "The skin still feels smooth and supple" | 4 | 5 | 21 |
| "The skin is dry again" | 23 | 2 | 5 |
| | | | |

| *After two days of using the cream* | | | |
|---|---|---|---|
| "I like the cream" | 1 | 7 | 20 |
| "The effect of the cream is long lasting" | 4 | 8 | 16 |

### A pilot study on children having atopic dry skin

The study was performed at two Swedish hospitals by dermatologists specialised in the field of atopic dermatitis. Twenty children were treated for two months with cream D described above. The age of the children varied between one and twelve years and they were all having widespread atopic dry skin, regularly developing into periods of acute atopic dermatitis. The dermatitis was treated in the normal way, i.e. with glucocorticoids of varying potencies. Treatment with cream D was started at a stage with no acute dermatitis and the cream was applied only once daily at bedtime.

Preliminary results strongly indicate the potential of cream D with respect to its ability to decrease frequency as well as seriousness of the periods of atopic dermatitis. Furthermore, the previously necessary amounts of glucocorticosteroids used could be significantly reduced if cream D was used to prevent skin from being dry and sensitive.

## Claims

1. Use of an oil-in-water emulsion, comprising an oily material, an aqueous phase and a galactolipid material as an emulsifier, as a carrier for a topical formulation for providing a prolonged local effect of an incorporated pharmaceutically or cosmetically active substance.

2. Use according to claim 1, wherein the formulation comprises 0.1-50 % by weight of oily material and 0.5-20 % by weight of emulsifier.

3. Use according to claim 1 or 2, wherein the formulation comprises 1-40 % by weight of oily material and 0.5-20 % by weight of emulsifier.

4. Use according to any of claims 1-3, wherein the galactolipid material consists of at least 50 % by weight of digalactosyldiacylglycerols and a remainder of other polar lipids, and constitutes an amount of 1.0-5.0 % by weight of the formulation.

5. Use according to any of claims 1-4, wherein the galactolipid material consists of 50-70 % by weight of digalactosyldiacylglycerols and 30-50 % by weight of other polar lipids.

6. Use according to any of claims 1-3, wherein the galactolipid material is a fractionated oat oil which consists of at least 15 % by weight of digalactosyldiacylglycerols and a remainder of other polar and non-polar lipids, and constitutes an amount of 2.0-10 % by weight of the formulation.

7. Use according to any of claims 1-3 and 6, wherein the galactolipid material is a fractionated oat oil which contains 40-60 % by weight polar lipids and a remainder of non-polar lipids.

8. Use according to any of claims 1-7, of a cream base, comprising, in % by weight
| | |
|---|---|
| Oily material | 10.0-30.0 % |
| Galactolipid emulsifier | 0.5-5 % |
| Thickener | 2.0-10.0 % |
| Preservative | 0.1-1.0 % |
| Water | ad 100 % |

9. Use according to any of clains 1-8 for the preparation of a topical cream or lotion, incorporating a moisturiser, especially glycerol, as the active substance.

10. Use according to any of claims 1-9 for the preparation of a medicament for prophylaxis or treatment of atopic dermatitis.

11. Use according to any of claims 1-8 for the preparation of a topical cream or lotion, incorporating a corticosteroid as the active substance, for treatment of skin inflammation.

12. Use according to any of claims 1-8, for the preparation of a topical anti-psoriatic cream or lotion, incorporating 13-hydroxy-linoleic acid as the active substance.

## Patentansprüche

1. Verwendung einer Öl-in-Wasser-Emulsion, die ein öliges Material, eine wässrige Phase und ein Galactolipidmaterial als Emulgator umfasst, als Träger für eine topische Formulierung zur Erzielung eines anhaltenden lokalen Effekts der Inkorporierung einer pharmazeutisch oder kosmetisch aktiven Substanz.

2. Verwendung gemäss Anspruch 1, worin die Formulierung 0,1-50 Gew.% öliges Material und 0,5-20 Gew.% Emulgator umfasst.

3. Verwendung gemäss Anspruch 1 oder 2, worin die Formulierung 1-40 Gew.% öliges Material und 0,5-20 Gew.% Emulgator umfasst.

4. Verwendung gemäss mindestens einem der Ansprüche 1 bis 3, worin das Galactolipidmaterial aus mindestens 50 Gew.% Digalactosyldiacylglycerolen und dem Rest aus anderen polaren Lipiden besteht und in einer Menge von 1,0-5,0 Gew.% zu der Formulierung beiträgt.

5. Verwendung gemäss mindestens einem der Ansprüche 1 bis 4, worin das Galactolipidmaterial zu 50-70 Gew.% aus Digalactosyldiacylglycerolen und zu 30-50 Gew.% aus anderen polaren Lipiden besteht.

6. Verwendung gemäss mindestens einem der Ansprüche 1 bis 3, worin das Galactolipidmaterial ein fraktioniertes Haferöl ist, das zu mindestens 15 Gew.% aus Digalactosyldiacylglycerolen und zum Rest aus anderen polaren und nicht-polaren Lipiden besteht und in einer Menge von 2,0-10 Gew.% zu der Formulierung beiträgt.

7. Verwendung gemäss mindestens einem der Ansprüche 1 bis 3 und 6, worin das Galactolipidmaterial ein fraktioniertes Haferöl ist, das 40-60 Gew.% polare Lipide und als Rest nicht-polare Lipide enthält.

8. Verwendung gemäss mindestens einem der Ansprüche 1 bis 7, in Form einer Cremebasis, die in Gew.% folgendes umfasst:
| | |
|---|---|
| öliges Material | 10,0-30,0 % |
| Galactolipid-Emulgator | 0,5- 5 % |
| Verdickungsmittel | 2,0-10,0 % |
| Konservierungsmittel | 0,1- 1,0 % |
| Wasser auf | 100 % |

9. Verwendung gemäss mindestens einem der Ansprüche 1 bis 8 zur Herstellung einer topischen Creme oder Lotion, die einen Feuchtigkeitsspender, insbesondere Glycerin, als aktive Substanz enthält.

10. Verwendung gemäss mindestens einem der Ansprüche 1 bis 9 zur Herstellung eines Medikaments zur Prophylaxe oder Behandlung von atopischer Dermatitis.

11. Verwendung gemäss mindestens einem der Ansprüche 1 bis 8 zur Herstellung einer topischen Creme oder Lotion, die ein Corticosteroid als aktive Substanz enthält, zur Behandlung von Hautentzündungen.

12. Verwendung gemäss mindestens einem der Ansprüche 1 bis 8 zur Herstellung einer topischen Antipsoriasis-Creme oder -Lotion, die 13-Hydroxy-linolsäure als aktive Substanz enthält.

## Revendications

1. Utilisation d'une émulsion huile-dans-eau, comprenant une matière huileuse, une phase aqueuse et un galactolipide en tant qu'émulsifiant, comme excipient pour une formulation topique pour fournir un effet local prolongé d'une substance incorporée active pharmaceutiquement ou cosmétiquement.

2. Utilisation selon la revendication 1, dans laquelle la formulation comprend de 0,1 à 50 % en poids de matière huileuse et de 0,5 à 20 % en poids d'émulsifiant.

3. Utilisation selon la revendication 1 ou 2, dans laquelle la formulation comprend de 1 à 40 % en poids de matière huileuse et de 0,5 à 20 % en poids d'émulsifiant.

4. Utilisation selon l'une quelconque des revendications 1 à 3, dans laquelle le galactolipide se compose d'au moins 50 % en poids de digalactosyl diacylglycérols et d'un résidu d'autres lipides polaires, et constitue une quantité de 1,0 à 5,0 % en poids de la formulation.

5. Utilisation selon l'une quelconque des revendications 1 à 4, dans laquelle le galactolipide se compose de 50 à 70 % en poids de digalactosyl diacylglycérols et de 30 à 50 % en poids d'autres lipides polaires.

6. Utilisation selon l'une quelconque des revendications 1 à 3, dans laquelle le galactolipide est une huile d'avoine fractionnée qui se compose d'au moins 15 % en poids de digalactosyl diacylglycérols et d'un résidu d'autres lipides polaires et non polaires, et constitue une quantité de 2,0 à 10 % en poids de la formulation.

7. Utilisation selon l'une quelconque des revendications 1 à 3 et 6, dans laquelle le galactolipide est une huile d'avoine fractionnée qui contient de 40 à 60 % en poids de lipides polaires et un résidu de lipides non polaires.

8. Utilisation selon l'une quelconque des revendications 1 à 7, d'une base de crème, comprenant, en % en poids
| | |
|---|---|
| Matière huileuse | 10,0 à 30,0 % |
| Emulsifiant à base de galactolipide | 0,5 à 5 % |
| Epaississant | 2,0 à 10,0 % |
| Conservateur | 0,1 à 1,0 % |
| Eau | jusqu'à 100 % |

9. Utilisation selon l'une quelconque des revendications 1 à 8, pour la préparation d'une crème ou lotion topique, incorporant une substance hydratante, en particulier le glycérol, en tant que substance active.

10. Utilisation selon l'une quelconque des revendications 1 à 9, pour la préparation d'un médicament destiné à la prophylaxie ou au traitement de la dermatite atopique.

11. Utilisation selon l'une quelconque des revendications 1 à 8, pour la préparation d'une crème ou lotion topique, incorporant un corticostéroïde en tant que substance active, pour le traitement des inflammations cutanées.

12. Utilisation selon l'une quelconque des revendications 1 à 8, pour la préparation d'une crème ou lotion topique antipsoriatique, incorporant de l'acide 13-hydroxylinoléique en tant que substance active.
